# EUROPEAN PATENT SPECIFICATION

(11) **EP 0 313 069 B1**
(45) Date of publication and mention of the grant of the patent: **20.01.1993**
(21) Application number: 88117569.9
(22) Date of filing: 21.10.1988
(51) Int. Cl.: B29C 39/42

(54) **Process for manufacturing spherical objects**
Verfahren zum Herstellen von sphärischen Gegenständen
Procédé pour la fabrication d'objets sphériques

(30) Priority: 23.10.1987 JP 268707/87
(43) Date of publication of application: 26.04.1989
(73) Proprietor: NIPPON ZEON CO., LTD., Chiyoda-ku Tokyo 100 (JP)
(72) Inventor: Nagase, Toshio, Tsukuba-gun Ibaragi-ken (JP)
(74) Representative: TER MEER STEINMEISTER & PARTNER GbR

(56) References cited:
- DE-A- 3 508 439
- US-A- 2 298 348
- US-A- 4 212 621
- US-A- 4 783 217
- PATENT ABSTRACTS OF JAPAN, vol. 11, no. 206 (C-433)[2653], 3rd July 1987; & JP-A-62 029 979 (TAKENAKA KOMUTEN CO., LTD) 07-02-1987

## Description

This invention relates to a process for manufacturing spherical objects, especially occluder for the ball valve as a component of an artificial heart.

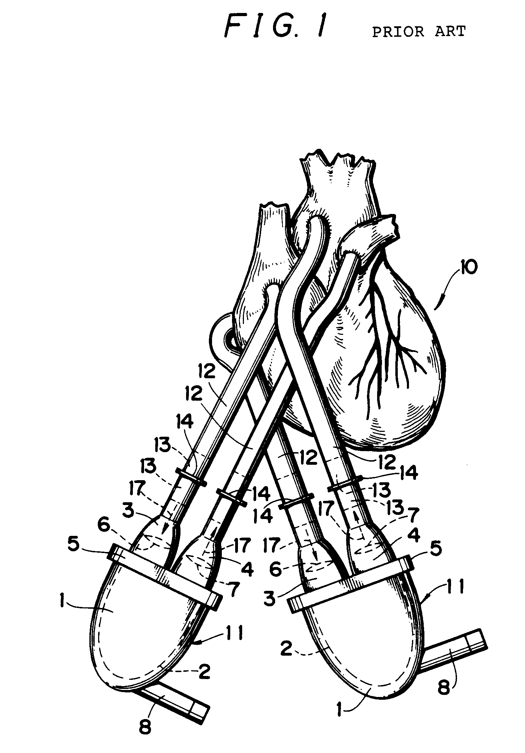

In recent years, increasing effort has been expended in the development of an extra- or paracorporeally-located artificial heart to assist cardiac function temporarily in place of the natural heart of a patient during open-heart surgery or the like. For example, referring to Fig. 1 in which a reference character 10 designates a heart. A pair of sac type blood pumps 11 are connected, one between the right atrium and the pulmonary artery and the other between the left atrium and the aorta. Such sac type blood pumps have been studied and applied as assistant heart to the clinical treatment in Japan as well, as forerunner in the world.

Blood pumps 11 of sac type are each composed essentially of a blood (pumping) chamber 2 and a pressure housing or outer case 1 which is, for example, made of polycarbonate or polyurethane resin and which envelop airtight blood chamber 2. Blood chamber 2 has on the top a pair of inflow and outflow ports 3, 4 which project upward substantially-parallel to each other and through which blood flows to or from the blood chamber. Housing 1 is flanged (the flange is designated at a reference numeral 5) to enclose airtight chamber 2 therein. Blood inflow and outflow ports 3, 4 each are fitted inside with an artificial check valve 6 or 7 in order to avoid backflow of blood 17, this assuring that blood 17 is introduced through blood inflow port 3 into chamber 2 and pumped out pulsatively through blood outflow port 4. Such pulsatile pumping of blood results from the repeated alternation of expansion and contraction caused by the alternative variation in outside pressure on chamber 2 in accordance with repeated alternation of introduction of compressed air into chamber 2 and suction of it therefrom through a single port 8 at the bottom of housing 1. Inflow port 3 is connected through a connector 13 to a cannula 12 anastomosed to the heart, each being inserted into the connector from opposite end to the other till the flanged center 14 thereof.

As a valve component 6, 7 of such blood pumps as above-stated are used, for example, disk or ball valve. The latter comprises a movable ball which contributes to great durability and small risk of thrombosis compared with the former. Both type valves however has difficulty of involving the disturbance of blood stream which is due to the provision of the strut and cage of the valve in blood stream. This can lead to thrombus formation.

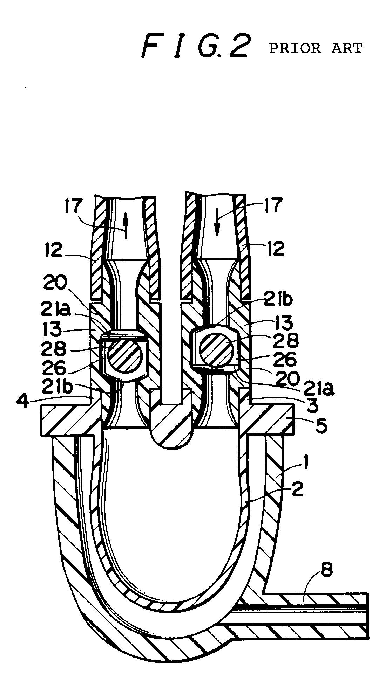

In Japanese Patent Publication No. 42759/1979 is disclosed a blood pump comprising ball valves without strut nor cage in question, as shown in Fig.2 in which a reference numeral 13 designates a connector. It has the following advantages:
(1) It can be manufactured in a simpler process with a smaller number of parts and with simpler and inexpensive molds.
(2) It has good response characteristic: valve ball can be instantly moved from one position where the ball is placed in point contact with a valve seat 21a to allow blood 17 to pass between ball 28 and the valve body 20 to the other position where the ball is placed in total contact with a valve seat 21b not to allow backflow of blood 17.
(3) It is made of plastic and rubber, and nothing else, and nevertheless has a relatively good durability.

The above-mentioned balls 28 are usually made of rubber such as silicone and the sphericity of them is most important for the ball valve. Inadequate sphericity reflects formation of clearance between the ball and the valve seat, resulting in malfunction to enough prevent backflow. As counter-measure against this, a ball obtained by casting, injection molding, or the like, is required to be surface-polished. In general however precise polishing of a formed rubber object can be accomplished with much difficulty.

An object of the present invention is to provide a process for manufacturing in a simplified way balls having a high sphericity and particularly suitable for use in ball valve.

The above-mentioned object is achieved by the process according to claim 1.

Other objects, features and advantages of the invention will appear more fully from the following detailed description thereof taken in connection with the accompanying drawings.
Fig.1 is schematically-illustrative diagram of a pair of prior art blood pumps connected to the heart;
Fig.2 is an elevational sectional view of another prior art blood pump;
Figs.3 through 8 are illustrative of embodiments of the present invention:

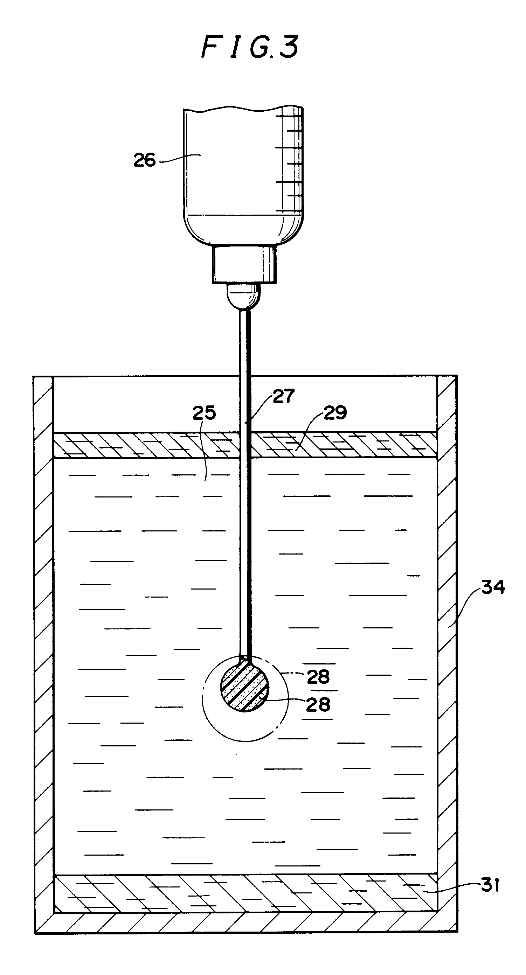

Fig.3 is a diagram in cross-sectional view illustrative of the process according to the invention for manufacturing balls;

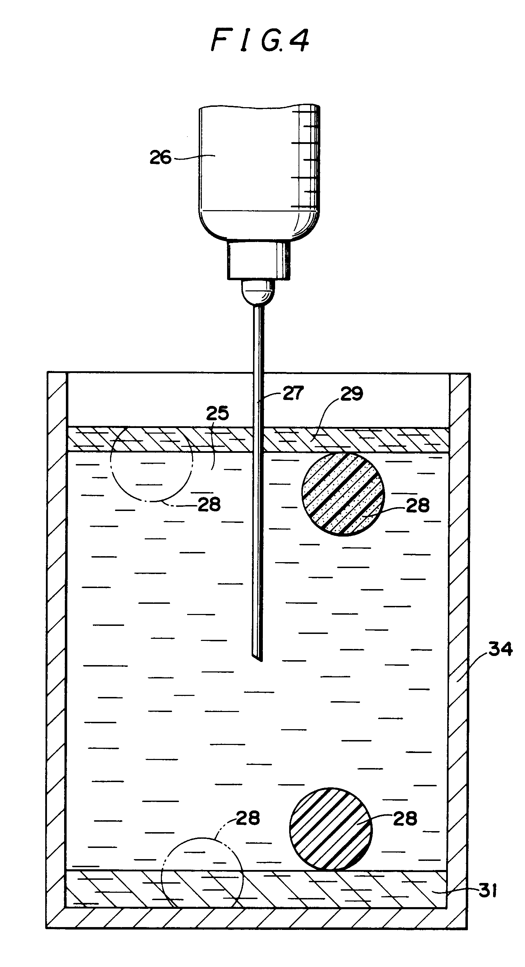

Fig.4 is a similar diagram illustrative of the course of making balls;

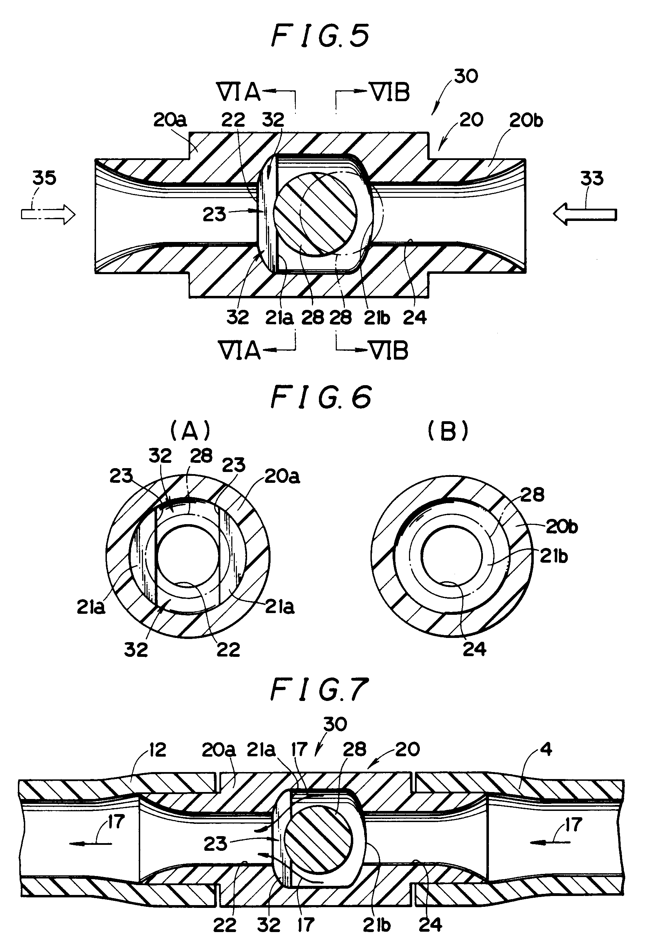

Fig.5 is a cross-sectional view of a valve provided with a ball made in the process illustrated in Figs.3 and 4;
Fig.6A is a cross-sectional view taken along line VIA-VIA of the Fig.5;
Fig.6B is a cross-sectional view taken along line VIB-VIB of the Fig.5;
Fig.7 is a schematically-illustrated diagram of the connection of the same valve; and

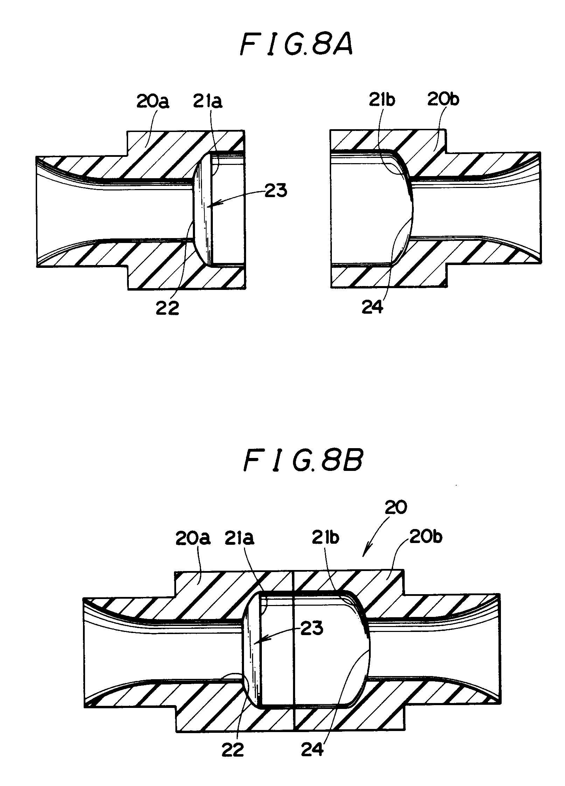

Figs.8A through 8C illustrates a series of steps of the procedure of assembling a similar valve.

The invention will be described more fully in the following:
With reference to Figs.3 and 4, the process of making valve balls according to the invention will be described.

As shown in Fig.3, a container 34 includes three (top, intermediate and bottom) layers 29, 25 and 31. The intermediate layer 25 in the container is of liquid having almost the same specific gravity as that of liquid material 28 from which valve balls are made. Suitable liquids as liquid 25 are, for example, protective colloids or surface active agents such as polyvinyl alcohol, carboxymethyl cellulose, polyvinyl pyrrolidone, sodium polyacrylate, methyl cellulose or the like, and those containing inorganic salt (such as sodium polyphosphate). For making a valve ball, liquid material 28 is gently downwardly ejected through a needle 27 by means of a syringe 26, such as microsyringe, attached to the needle into the intermediate liquid layer (or medium) 25 maintained at a constant temperature in order to prevent convection. Intermediate liquid layer 25 is desired to be kept within the temperature range of 40 to 80°C from the viewpoint of promoting thermal condensation, polycondensation or reaction, and may be kept at higher temperature unless convection is caused. Suitable examples of liquid material 28 to be ejected include two-liquid, room-temperature-vulcanization monomers such as silicone solution, or other monomer solutions such as for polyurethane, epoxy resins (with mediums other than water), etc.

The top layer 29 is of a liquid having a smaller specific gravity than that of the intermediate liquid layer 25 and lying the latter. The layer 29 is preferably water-soluble has a boiling point over 60 to 70°C. Suitable examples as liquids of the top layer 29 include alcohols such as isopropyl alcohol and butyl alcohol. Other liquids may be used if smaller in specific gravity than water. For the bottom layer 31 having a greater specific gravity than that of the layer 25 may be used water-soluble liquid, for example, glycerin, or the like. It is a matter of course that liquids of both layers 29, 31 are desirable to be those in which valve ball material 28 is insoluble.

A predetermined volume of solution 28 ejected through needle 27 floats as a mass in intermediate layer 25 during which it is subjected to reaction such as thermal condensation or polycondensation to be set, with increasing specific gravity. The specific gravity of the mass 28 is lower immediately after ejected, and with the lapse of time reaches the same as the specific gravity of the intermediate layer 25. Under conditions around this, like zero-gravity condition, the mass 28, while becoming substantially stationary, undergoes uniform pressure around the overall periphery thereof, this contributing to the formation into a ball of a predetermined diameter and having a high sphericity as indicated by a dash-and dot line in Fig.3.

Upon being ejected into the intermediate liquid layer (or medium) through needle 27 as above-stated, a substantially spherical mass of solution 28 is formed and begins to rise gradually in the medium because the solution 28 is a monomer having a lower specific gravity (smaller than 1) than that of intermediate liquid layer, and is stopped against the undersurface of top layer 29 as indicated with solid line in Fig.4. Without the top layer 29, the spherical mass 28 would be exposed to the air as indicated by a dash-and-dot line and readily get burst by the atmospheric pressure.

During floating, the spherical mass 28 continues reaction (thermal setting) with gradually increasing specific gravity, and when its specific gravity reaches the same as the specific gravity of the intermediate liquid layer 25, it becomes stationary and undergoes uniform pressure around the overall periphery, like zero gravity condition, this contributing to most higher sphericity of the mass 28. Further polymerization of spherical mass 28 is accompanied by increasing specific gravity (greater than 1) of it beyond that of the intermediate liquid layer 25, and thus spherical mass 28 begins to sink as indicated by solid line in Fig.4. Upon sinking against the surface of bottom liquid layer 31 having a greater specific gravity than those of the other layers, spherical mass 28 is repulsed resiliently by the cushion-like work of the bottom liquid layer 31. In the course as above-stated, thermal setting of spherical mass 28 is completed with high sphericity maintained.

Without bottom liquid layer 31, spherical mass (ball) 28 collides against the hard bottom of the container 34 to become deformed as indicated by a dash-and-two-dot line, resulting in decrease of sphericity.

As described above, the process according to the present invention assures to make valve balls 28 of high sphericity. The thus-obtained balls have so smooth surface that polishing is unnecessary. The provision of top and bottom liquid layers contributes to make very easy the practice of the process for making balls because otherwise control of the specific gravity of the spherical mass (solution 28) is extremely difficult.

Fig.5 shows a valve 30 for blood pump comprising the valve ball 28 installed therein which is made in the above-stated process consisting of ejection of material to be formed into the ball and reaction (thermal setting ) of the material while floating in substantially stationary state, thus without a special apparatus. When blood is flowing in the direction directed by arrow 33, ball 28 is forced against a valve seat 21a. The width of a groove 23 of the valve seat is smaller than the diameter of the ball 28 which can be inserted only partially as indicated by phantom line in Fig.6A and by solid line in Fig.3. Thus in the groove 23 remains a gap 32 connecting passage 22 between ball 28 and valve body 20a, thus ball 28 being place in substantially point contact with valve seat 21a.

On the other hand, if the backflow of blood in the direction indicated by arrow 35 occurs, ball 28 is moved to the position indicated with dash-and-dot line and forced against another circular valve seat 21b with the periphery of ball 28 fitting closely along the overall inner circumstance of seat 21b, consequently passage 24 being completely closed. Thus the backflow of blood can be totaly blocked.

Fig.7 shows the connection of a cannula 12 and a blood port through the above-mentioned valve 30, which corresponds to the integral unity of connector 13 and check valve in Fig.2. Ball 28 is moved in response to blood flow indicated by arrow 17 to valve seat 21a to be forced against it. In this case, blood is allowed to smoothly flow through passage 22 to the cannula 12. As understood, this valve built-in connector needs no installation of a check valve in blood flow port 3 or 4, thus permitting easy mounting of the valve.

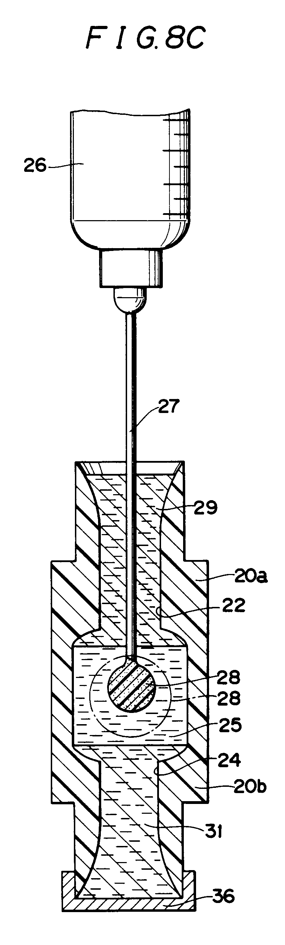

Fig.8 is illustrative of an alternative embodiment of valve.

In Fig.8A, the valve consists of two components 20a and 20b. These may be made by known injection molding technique from solid or plasticized polyvinyl chloride, polycarbonate, acrylic resin, etc., and they can be coated with known antithrombogenetic agent, such as polyurethane and siloxanes, and have transparency for making observation of the inside easy.

As shown in Fig.8B, valve components 20a and 20b may be joined together with adhesive or integrated together by means of a projecting part-corresponding cut-out space joint (not shown). Besides, the inner surface of the integrated valve is treated with antithrombogenetic agent (coated with antithrombogenetic agent), followed by drying, with an effect to obtain adequately smooth and antithrombogenetic film-formed inner surface.

As illustrated in Fig. 8C, a valve body capped (a reference numeral 36 designates a cap) at the bottom is put in vertical position. Into the valve body 20 are poured in sequence liquids 31, 25 and 29.

Under these conditions, in the same way as described in Fig.3, solution 28 is ejected to create a spherical mass, rise of which furing initial polymerization is blocked by liquid layer 29, and sinking of which after considerably having polymerized is blocked by liquid layer 31, thus a ball 28 having a high sphericity being formed in medium 25. The blocking liquid layers 29, 31 are preferably located at levels indicated in Figure 8C in order to avoid as possible the collision of the ball with the inner wall surface of valve body 20. In this way, installation of the ball 28 in valve body 20 is easy, and besides a ball 28 having an any intended size and a good sphericity can be made by controlling the volume of ball material to be ejected.

In addition, in this process, a valve ball can be installed in a valve body 20 which has been already finished, for example, the inner surface of the latter being possible to be previously coated with an antithrombogenetic agent, or the like, or processed otherwise. In virtue of these, ball valves having smoothed inner surface and adequately protective against thrombus formation can be provided.

It will be evident that various modification can be made to the described embodiments without departing from the scope of the present invention.

For example, blocking liquid layers 29, 31 may be changed differently in kind and specific gravity of liquid and in way of forming them in accordance with medium 25. Liquid material 28 from which valve balls are made may be of different specific gravity, and a spherical mass of material 28 may be produced in medium 25 in different ways. At least one of blocking liquid layers may be located in a suitable depth in medium layer 25 and may be composed of a plurality of liquids. The valve can be modified in structure, shape, size, etc. The above-mentioned groove 23 is not always necessary to be formed. Formation of valve balls may be carried out under a constant gravity though it is preferred to be carried out under zero gravity-like condition. Different liquids may be used as medium 25. The valve can be used in ways other than the above-stated.

As described above, the present invention has a feature that liquid ball material is subjected to reaction to be set while floating in the medium between top or upper and bottom or lower blocking liquid layers, and thereby, as stated above, balls having high sphericity can be always obtained without undergoing burst or deformation. In the process according to the present invention, the formed balls have so smooth or mirror-like surface that polishing is unnecessary, and the formation of the valve balls requires to be carried out while floating in a medium, and nothing else, and thus can be accomplished very easily without needing special apparatus or equipment.

## Claims

1. A process for manufacturing spherical objects comprising ejecting into a reaction medium layer a predetermined amount of liquid material to be made into spherical objects and allowing the spherical mass produced by the ejection to react to be set while floating in said reaction medium layer, said reaction medium layer being restricted by a blocking and protective liquid layer having a smaller specific gravity than that of said reaction medium layer and located above the level of the ejection and another blocking and protective liquid layer having a greater specific gravity than that of said reaction medium layer and located below said level of the ejection.

2. A process as claimed in claim 1 wherein said liquid medium layer is of almost the same specific gravity as that of said liquid material to be formed into spherical objects, said blocking and protective liquid layer having a smaller specific gravity than that of said reaction medium layer overlies said reaction medium layer, and said blocking and protective liquid layer having a greater specific gravity than that of said reaction medium layer underlies said reaction medium layer.

3. A process as claimed in claim 2 wherein said liquid material to be formed into spherical objects is of monomer solution, said reaction medium layer is of polymer-containing liquid, said blocking and protective liquid layers are water-soluble, and said liquid material to be formed into spherical objects is insoluble in said blocking and protective liquid layers.

4. A process as claimed in claim 1 wherein said liquid material is ejected into said reaction medium not so as to produce convection while said reaction medium is maintained at reaction-promoting temperature.

5. A process as claimed in claim 1 wherein spherical occluders for ball valves for use in an artificial heart are made.

6. A process as claimed in claim 5 wherein a spherical occluder is made in a ball valve body, comprising the steps of pouring in sequence said blocking and protective liquid layer having a greater specific gravity, said reaction medium and said blocking and protective liquid layer having a smaller specific gravity into said valve body, and ejecting said liquid material into said reaction medium.

7. A process as claimed in claim 5 wherein said ball valve is used as a connector.

## Patentansprüche

1. Verfahren zur Herstellung sphärischer Gegenstände, umfassend das Ausstoßen einer vorbestimmten Menge eines flüssigen Materials, aus welchem sphärische Gegenstände hergestellt werden sollen, in eine Reaktionsmediumsschicht und Reagierenlassen der durch die Ausstoßung erzeugten sphärischen Masse, um gehärtet zu werden, während sie in der Reaktionsmediumsschicht schwimmt, wobei die Reaktionsmediumsschicht begrenzt wird durch eine sperrende und schützende Flüssigkeitsschicht, die ein Kleineres spezifisches Gewicht als das der Reaktionsmediumsschicht besitzt und oberhalb des Niveaus der Ausstoßung angeordnet ist sowie eine weitere sperrende und schützende Flüssigkeitsschicht, welche ein größeres spezifisches Gewicht als das der Reaktionsmediumsschicht aufweist und unterhalb des Niveaus der Ausstoßung angeordnet ist.

2. Verfahren nach Anspruch 1, wobei die Flüssigmediumsschicht nahezu das gleiche spezifische Gewicht wie das des flüssigen Materials, aus welchem sphärische Gegenstände hergestellt werden sollen, aufweist, die sperrende und schützende Flüssigkeitsschicht mit einem Kleineren spezifischen Gewicht als dem der Reaktionsmediumsschicht die Reaktionsmediumsschicht überlagert und die sperrende und schützende Flüssigkeitsschicht mit einem größeren spezifischen Gewicht als dem der Reaktionsmediumsschicht unterhalb der Reaktionsmediumsschicht liegt.

3. Verfahren nach Anspruch 2, wobei das flüssige Material, aus welchem sphärische Gegenstände hergestellt werden sollen, eine Monomerlösung ist, die Reaktionsmediumsschicht aus einer Polymer enthaltenden Flüssigkeit besteht, die sperrenden und schützenden Flüssigkeitsschichten wasserlöslich sind und das flüssige Material, aus welchem sphärische Gegenstände hergestellt werden sollen, in den sperrenden und schützenden Flüssigkeitsschichten unlöslich ist.

4. Verfahren nach Anspruch 1, wobei das flüssige Material derart in das Reaktionsmedium ausgestoßen wird, daß keine Konvektion erzeugt wird. während das Reaktionsmedium bei einer die Reaktion fördernden Temperatur gehalten wird.

5. Verfahren nach Anspruch 1, wobei sphärische Okkludiervorrichtungen für Kugelventile zur Verwendung in einem Kunstherz hergestellt werden.

6. Verfahren nach Anspruch 5, wobei eine sphärische Okkludiervorrichtung in einem Kugelventilkörper hergestellt wird, umfassend die Stufen des aufeinanderfolgenden Eingießens der sperrenden und schützenden Flüssigkeitsschicht mit einem größeren spezifischen Gewicht, des Reaktionsmediums und der sperrenden und schützenden Flüssigkeitsschicht mit einem kleineren spezifischen Gewicht in den Ventilkörper sowie des Ausstoßens des flüssigen Materials in das Reaktionsmedium.

7. Verfahren nach Anspruch 5, wobei das Kugelventil als Verbindungseinrichtung verwendet wird.

## Revendications

1. Procédé de fabrication d'objets sphériques, consistant à éjecter, dans une couche de milieu de réaction, une quantité prédéterminée d'une matière liquide destinée à être formée en objets sphériques et à laisser réagir la masse sphérique produite par l'éjection, de façon qu'elle durcisse alors qu'elle flotte dans la couche de milieu de réaction, cette dernière étant maintenue par une couche de liquide de protection et de blocage, ayant une densité inférieure à celle de la couche de milieu de réaction et située au-dessus du niveau de l'éjection, et une autre couche de liquide de protection et de blocage, ayant une densité supérieure à celle de la couche de milieu de réaction et située au-dessous du niveau de l'éjection.

2. Procédé suivant la revendication 1, selon lequel la couche du milieu liquide a presque la même densité que celle de la matière liquide devant être formée en objets sphériques, tandis que l'a couche de liquide de protection et de blocage ayant une densité supérieure à celle de la couche de milieu de réaction est située au-dessus de cette dernière et que la couche de liquide de protection et de blocage ayant une densité supérieure à celle de la couche de milieu de réaction est située au-dessous de cette dernière.

3. Procédé suivant la revendication 2, selon lequel la matière liquide destinée à être formée en objets sphériques est une solution monomère, la couche de milieu de réaction est un liquide contenant un polymère, les couches de liquide de protection et de blocage sont solubles dans l'eau et la matière liquide destinée à être formée en objets sphériques est insoluble dans les couches de liquide de protection et de blocage.

4. Procédé suivant la revendication 1, selon lequel la matière liquide est éjectée dans le milieu de réaction d'une manière ne produisant pas de convection, tandis que le milieu de réaction est maintenu à une température favorisant la réaction.

5. Procédé suivant la revendication 1, selon lequel on réalise des obturateurs sphériques pour des clapets à bille destinés à être utilisés dans un coeur artificiel.

6. Procédé suivant la revendication 5, selon lequel on réalise un obturateur sphérique dans un corps de clapet à bille en exécutant les opérations consistant à verser successivement, dans le corps de clapet, la couche de liquide de protection et de blocage ayant une densité supérieure, le milieu de réaction et la couche de liquide de protection et de blocage ayant une densité inférieure, puis à éjecter la matière liquide dans le milieu de réaction.

7. Procédé suivant la revendication 5, selon lequel le clapet à bille est utilisé en tant que raccord.
